# EUROPEAN PATENT SPECIFICATION

(11) **EP 1 656 165 B1**
(45) Date of publication and mention of the grant of the patent: **17.12.2008**
(21) Application number: 04768107.7
(22) Date of filing: 18.08.2004
(51) Int. Cl.: A61L 27/34, A61L 27/54, A61L 29/04, A61L 31/10, A61L 31/16, A61F 2/06

(54) **POLYMERIC DRUG RELEASE SYSTEM FOR MEDICAL DEVICES**
POLYMERISCHES ARZNEIMITTELFREISETZUNGSSYSTEM FÜR MEDIZINISCHE GERÄTE
SYSTEME POLYMERE DE LIBERATION DE MEDICAMENT POUR DISPOSITIFS MEDICAUX

(30) Priority: 19.08.2003 GB 0319461; 27.10.2003 GB 0325060
(43) Date of publication of application: 17.05.2006
(73) Proprietor: PolyBioMed Limited, Abingdon, Oxfordshire OX14 4RX (GB)
(72) Inventor: AI-LAMEE, Kadem, Polybiomed Limited, Sheffield S9 3SP (GB); LOTT, Martyn, Polyiomed Limited, Sheffield S9 3SP (GB)
(74) Representative: Tollett, Ian
(86) International application number: PCT/GB2004/003547
(87) International publication number: WO 2005/018696

(56) References cited:
- EP-A- 0 153 863
- WO-A-01/87372
- WO-A-98/55162
- WO-A-98/58690
- WO-A-02/055122
- WO-A-03/024500
- US-A- 5 968 538

## Description

The present invention relates to a coating composition for a medical device, a method of coating a medical device and a device coated with the composition. In particular, it relates to the incorporation of more than one bioactive agent into a coating composition and a method for using the composition to coat an implantable medical device.

It is known to apply biologically active materials to the surface of an implantable medical device such as a stent, see for example WO 03/024500.

The application of drug combinations to stents is also disclosed in WO 01/87372 (Cordis Corporation). Example 6 relates to spraying a stent with a solution of poly (ethylene-covinyl acetate), polybutyl methacrylate and rapamycin dissolved in tetrahydrofuran followed by spraying the thus-coated stent with a solution of poly (ethylene-co-vinyl acetate) polybutyl methacrylate and dexamethasone dissolved in tetrahydrofuran.

US 6,258,121 and US 6,569,195 (both SciMed Life Systems, Inc.) disclose a polymeric coating for a stent comprising a blend of a faster releasing hydrophilic polymeric material (such as a polylactic acid/polyethelene oxide copolymer) and a second slower releasing hydrophobic material (such as a polylactic acid/polycaprolactone copolymer). This polymeric blend can be combined with an active agent such as Taxol which is delivered from the stent to inhibit restenosis following angioplasty. However one of the problems with the disclosed coating is that polylactic acid is biodegradable.

A further multi-layer polymer coating for a vascular stent is disclosed in WO 02/074194 (STS Biopolymers, Inc.). As with the SciMed references, this coating can include a slow drug release layer and a fast drug release layer, with the drug release rate profile being adjustable by adjusting the ratio of hydrophilic to hydrophobic polymers.

WO 02/055122 (Biocompatibles Limited) discloses an amphiphilic polymer coating for a vascular stent loaded with a sparingly water soluble restenosis inhibiting drug.

WO 03/018083 (Chosun University et. al.) discloses another covering composition for a drug-release stent which includes polyuretheyne, polyethelyne glycol, a drug and an organic solvent.

WO 03/035135 (Scimed Life Systems Inc.) discloses another coating composition for a drug- release stent which includes a styrene-isobutylene based block copolymer, paclitaxel and organic solvent. The drug release profile for this coating is dependant upon the drug-to-polymer ratio. Thus, the slow release formulation has a three-fold greater coating weight compared with the moderate release formulation.

The present invention seeks to provide an improved release system for implantable devices. Preferred aspects of the present invention seek to provide a system in which drugs or other biologically active materials are released in a controlled and programmable manner, i.e. at a desired rate and/or over a desired period of time and/or after a predetermined period of time after implantation of the device.

According to a first aspect of the present invention, there is provided a coating composition for a medical device comprising a combination of a bioactive and a vehicle therefor, wherein the vehicle comprises a first compound which is a random copolymer of Formula . 1:

[A]ₓ₋[B]_{y}-[C]_{z}

wherein A is a vinyl acetal group, B is a vinyl alcohol group and C is a vinyl acetate group and wherein x, y and z represent the proportions of the groups A, B and C in the copolymer so that x+y+z=1, and x is from 0.8 to 0.9, y is from 0.1 to 0.2 and z is from 0 to 0.025.

In a particularly preferred embodiment, the vehicle additionally comprises a second compound which is a polymer of Formula 2:

[D]ₙ-[E]ₘ

wherein D is a vinyl pyrrolidone group and E is a vinyl acetate group and wherein n and m represent the proportions of the groups D and E in the copolymer so that n+m=1. It has been found that a useful composition results from employing only vinyl pyrrolidone as the second compound, and therefore m can be zero in Formula 2 above. Preferably, however, n is from 0.3 to 0.7 and m is from 0.3 to 0.7.

The proportion of the second compound may be over 50% by weight of the coating composition.

In a preferred embodiment, [A]ₓ₋[B]_{y}-[C]_{z} is a compound of Formula 1A: 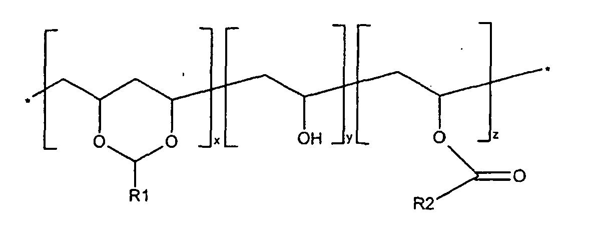 wherein R1 and R2 are independently H or an alkyl, alkenyl, alkynyl or aryl group and wherein optionally an alkyl, alkenyl, alkynyl or aryl group may be substituted for any pendent hydrogen atom. More preferably, R1 and R2 are independently C1-C6 alkyl.

Although the first compound can be bought "off the shelf", it can also be synthesised from vinylacetate (CH₂CHOCOCH₃). This is hydrolysed to form one of the co-polymers (polyvinylalcohol), reacts with an aldehyde (butyl-1-al in the preferred embodiment) to form the co-polyvinylacetal co-polymer and itself forms the co-vinylacetate co-polymer.

In the particularly preferred embodiment, the first compound is a polymer which is poly(vinylbutyral-co-vinyl alcohol-co-vinyl acetate) with an average Mw from 50,000 to 80,000 and with 88 wt% vinyl butyral groups: 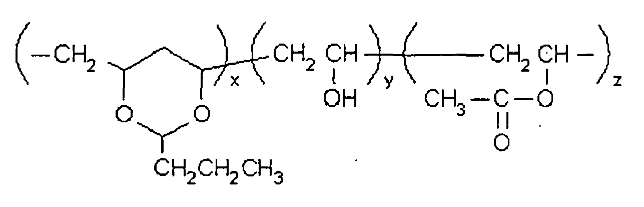 which is commercially available from Solutia under the trade mark Butvar® and the second compound is a polymer which is poly(vinyl pyrrolidone-co-vinyl acetate) with an average M_{w} of 50,000:

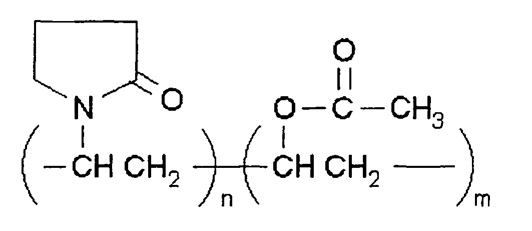

The present applicant has discovered that employing a copolymer of Formula 1 provides an effective vehicle for a bioactive to be released from a medical device such as a stent. In particular, the exemplary copolymer PVB combines hydrophobicity with good adhesion properties.

The applicant has further discovered that an especially effective vehicle can be provided by combining the copolymer of Formula 1 with the copolymer of Formula 2 as defined above. These two classes of copolymers have been found to be particularly compatible and therefore suitable for use together in the inventive coating composition. The advantages of the combination include the following:
1. The glass transition temperatures (T_{g}) are very similar for both classes of copolymers. For example, the exemplary first copolymer PVB has T_{g} from 62 to 72 °C and the exemplary second copolymer PnVPA has T_{g} = 64°C.
2. The presence of acetate groups in both copolymers means that the copolymers are more likely to be miscible, and therefore there is less likely to a problem of phase separation.
3. The two classes of copolymers are soluble in similar solvents and a simple solvent system can be used therefore to dissolve and apply the coating composition, such as tetrahydrofuran, dichloromethane or chloroform. This is particularly important when it comes to using the copolymer vehicle to carry a bioactive, because many bioactives are temperature-sensitive and cannot therefore be employed in a method in which the drying of the composition on the medical implant takes place at high temperatures. With the present system, a low boiling point solvent such as chloroform can be used (with T_{b} = 60°C) and drying can therefore take place at 40°C under vacuum.
4. The copolymer combination has a wide range of solubility and can therefore be used with a wide range of bioactives.

The good compatibility of the preferred copolymer combination is in contrast to other combinations that have been tried such as PVB and polyethylene glycol or PVB with a combination of polyethylene glycol and polypropylene glycol, both of which result in compatibility problems.

A further advantage of the inventive composition is that it allows greater control and selectivity of the drug release than prior art compositions. For example, many prior art compositions release the drug too quickly for it to have the required effect, and therefore drug release is controlled by the use of polymer-only top coatings or variations in the polymer:drug ratio of the coating. In particular, the latter can lead to a requirement for coating thicknesses which may compromise coating integrity.

Other compositions, such as that disclosed in the Cordis reference mentioned above (WO 01/87372), attempt to provide a system which releases two different drugs at different rates with each drug targeting a different region of the wound healing response. However, the release profiles of the two drugs are in fact fairly similar, which means that the selectivity aimed for is not achieved. The present composition is far more effective at providing a system with distinct and selective release profiles.

According to a second aspect of the present invention, there is provided a method for coating a medical device comprising the step of:
(a) applying to at least a part of the device a first coating composition as defined above.
   In a particularly preferred embodiment, the method additionally comprising the step of:
(b) applying a second coating composition as defined above
wherein said first and said second coating composition are the same or different.

Both first and second coatings have vehicles comprising said first component and said second component in ratios which may range from 50:50 to 100:0. The composition of the first and second coatings depend on the properties and targeted release kinetics of the individual drugs. Hence the compositions can be the same or different.

The first coating preferably has a vehicle comprising said first compound and said second compound in a ratio from 80:20 to 100:0 (most preferably 98:2) and the second coating preferably has a vehicle comprising said first compound and said second compound in a ratio from 70:30 to 94:6 (most preferably 90:10) .

Although the bioactive in the second coating may be the same as that in the first, in the preferred embodiment different bioactives are employed in order to result in different effects *in vivo* as the bioactives are released over time. For example, the first coating composition may include an anti-proliferative agent such as rapamycin and the second coating may include an anti-inflammatory agent such as dexamethasone.

Other bioactives that can be employed include estradiol, taxol, vincristine, prostaglandins, vinblastine, heparin, or a nitric oxide donor.

The proportion of bioactive to vehicle may be typically from 1:9 to 1:1 and is preferably frog 1:4 to 1:2.

The coatings are preferably applied by spraying or dipping, and the second coating is preferably applied over part or all of the first coating.

According to a third aspect of the present invention, there is provided a medical device (such as a stent or graft-stent) which has been coated with a coating composition by means of a method as defined above. The stent is preferably be formed of a metal but the inventive compositions can adhere to many other materials such as PET, PTFE, nylon, polycarbonate, polypropylene and polyurethane.

Depending on the polymer and drug ratios required, adhesion to the surface can be enhanced by means of the method disclosed in WO 03/024500 (in the name of the present applicant), the contents of which are incorporated herein by reference. Where 100% PVB is used, application directly to an unmodified surface may be sufficient (although adhesion may optionally be enhanced by modification techniques). As the amount of PnVPA is increased in the formulation, surface modification may be required to maintain adequate adhesion.

In yet a further aspect of the present invention there is provided an implantable medical device having thereon a first coating of a first biologically active material in a first carrier material and a second coating of a second biologically active material in a second carrier material. The two carrier materials are preferably mixtures of polymers and may comprise the same two polymers in different proportions by weight. The proportions by weight may be selected so that the second biologically active material is released relatively quickly and the first biologically active material is released in a controlled manner and/or relatively slowly. In one embodiment, the carrier material is a single polymer, the other carrier material being a mixture of this polymer with another polymer.

Preferred embodiments of the present invention will now be described, by way of example only, with reference to the accompanying drawings, of which:
Fig. 1 is an enlarged sectioned view of part of a stent in accordance with a first embodiment of the present invention;
Fig. 2 to 4 are similar views, respectively, of stents in accordance with second, third and fourth embodiments of the present invention;
Figures 5 to 8 are drug elution profiles for stents which have been coated with compositions in accordance with the present invention where the drugs in question are dexamethasone, rapamycin, 17β-estradiol and dexamathasone/rapamycin (dual elution) respectively;
Figure 9 is a schematic representation of pull-off test equipment; and
Figure 10 is a graph showing the average adhesion strength for various test samples.

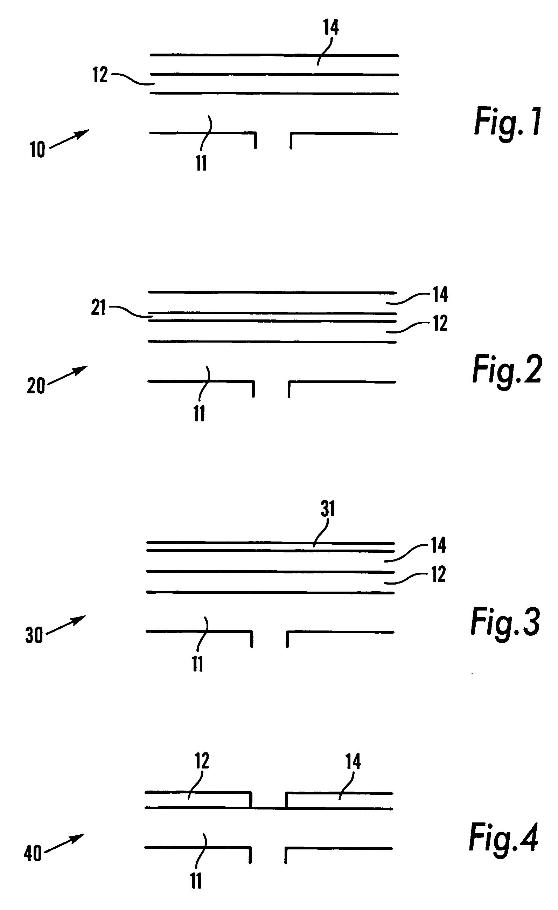

Referring to the drawings, Fig. 1 shows part of the wall 11 of a stent 10 of metallic material. To solve clinical problems, such as restenosis, produced by the implanting of the stent 10, it is treated as follows.

It is coated with a first layer 12 by spraying or dipping with rapamycin in a mixture of the two polymers poly(vinyl butyral-co-vinyl alcohol-co-vinyl acetate) (PVB) and poly (1-vinylpyrrolidone-co-vinyl acetate) (PnVPA).

PVB is predominantly hydrophobic and comprises the bulk of the formulation, preferably anything from 70% to 100%. Small additions of the hydrophilic PnVPA give the coating its "programmability" in terms of being able to control the release rate of drugs.

Layer 12 typically has a thickness of 6-7 microns. The rapamycin is provided in a suitable solvent which, after the application of layer 12, is removed by allowing the coating to dry. The drying operation also serves to maintain the activity of the rapamycin.

The stent is then similarly coated until a second layer 14 comprising dexamethasone in a mixture of the same two polymers. In this layer, the polymers PVB and PnVPA have different proportions by weight than in layer 12. Layer 14 typically has a thickness of 3-4 microns. The dexamethasone is provided in a suitable solvent which, after application of layer 14, is removed by drying.

The formulations of layers 12 and 14, and in particular the ratios of the polymers PVB and PnVPA, are selected to release their active agents at respective desired rates. As the dexamethasone is in the outer layer 14, it will be released first into the patient's body. The ratio of PVB, which is hydrophobic, to PnvPA, which is hydrophilic, is selected so that the dexamethasone is substantially released during the first few hours after implantation to reduce inflammation. Release of the dexamethasone, normally at a reducing rate, may continue for a period of up to ten days. Typical ratios for PVB to PnVPA in layer 14 are between 70:30 and 94:6, preferably 90:10.

The rapamycin in the lower layer 12 is released more slowly. Typical percentages by weight of PVB are 80% to 100%, i.e. layer 12 may be solely PVB with no PnVPA content. The preferred ratio of PVB to PnVPA is 98:2. The rapamycin is typically released over a period of ten days or more. An advantage of the above-described arrangement is that it achieves a selective-release and the right dose of a bioactive agent from each layer of the coating composition over a specified period of time. By independently varying the compositions of the polymer mixtures, the thicknesses of the layers and the concentration of the biologically active materials in the respective layers, the selective release of the active materials can be carefully controlled with reference to rate of release, duration of release and timing of initiation of release.

Various modifications may be made to the above-described arrangement. For example, the thickness of layers 12 and 14 may vary between 0.1 and 10 microns and may have different values in a single stent.

Instead of rapamycin, they may be used as the anti-proliferative agent estradiol, taxol, vincristine, vinblastine, or a nitric oxide donor. Appropriate mixtures of drugs may be incorporated in each of layer 12 and 14.

A primer layer (not shown) may be applied to the surface of stent 10 before the application of layer 12.

Other carrier materials may be used instead of the polymer mixtures disclosed above.

Preferably the entire surface of stent 10 is coated, but if desired parts of its surface may remain uncoated.

Preferably layer 14 covers all layer 12, but to achieve certain patterns of release, parts of layer 12 may remain uncoated.

Figure 2 shows a stent 20 in accordance with a second embodiment of the present invention in which an intermediate layer 21 in provided between layer 12 and 14. Layer 21 comprises a drug-free mixture of the polymers PVB and PnVPA which introduces an additional delay before the active material is released from layer 12. Typically the proportion of PVB is higher in layer 21 than in both layers 12 and 14, but this is not essential. To achieve certain patterns of release the proportion of PVB in layer 12 may be lower than in layer 14.

As shown in Figure 3, a stent 30 in accordance with a third embodiment has a barrier layer 31 provided over layer 14. Layer 13 serves to protect the underlying coatings. In a modification, a stent may have both an intermediate layer 21 and a barrier layer 31.

In a stent 40 according to a fourth embodiment of the present invention, to achieve a different pattern of release, layers 12 and 14 are applied to different regions of the wall 11 of the stent.

The stents may be made of a plastics material.

The features and modifications of the various embodiments may be interchanged or combined as desired.

The drug-eluting coatings disclosed can be used with coronary, peripheral or gastrointestinal stents or with other types of devices such as abdominal aortic aneurysm devices, anastomosis devices, heart valve repair devices, implantable biosensors, pacing and electro stimulation leads, vascular grafts or vena cava filters.

Other drugs may be used instead of those disclosed, including anti-platelet agents e.g prostaglandins and/or anti-coagulants agents e.g. heparin.

To release three or more biologically active materials, implantable devices are coated with three or more layers and such arrangements combine as desired the features of the various described embodiments.

### EXAMPLES

### Main Materials used

Poly(vinylbutyral-co-vinyl alcohol-co-vinyl acetate), average Mw 50,000 to 80,000. 88wt% vinyl butyral (referred to as PVB)

Poly(vinyl pyrrolidone-co-vinyl acetate), average Mw 50,000 (referred to as PnVPA)

N-[3-(trimethoxysilyl)propyl] ethylenediamine, 97%

3-(triethoxysilyl)propyl] isocyanate

All material supplied by Sigma-Aldrich Company Ltd

### Example 1: Dexamethasone

This example describes the preparation and elution of stents comprising various ratios of PVB/PnVPA and dexamethasone

Stents were cleaned by immersion in IPA for 30 mins with ultrasound and dried at 100°C overnight. All stents then underwent a surface modification procedure as outlined in WO03024500 by the same applicant.

Four coating solutions were prepared in a solvent blend of chloroform and acetone (ratio 1:7.4 by weight) comprising various ratios of PVB/PnVPA (Table 1). Dexamethasone was added to each formulation so that in terms of total solids 20% was dexamethasone, 80% polymer.

**Table 1**

| Coating Solution | Ratio PVB/PnVPA | Nomenclature | Ratio Polymer: Drug | Coating Solution Total Solids |
|---|---|---|---|---|
| 1 | 100:0 | PEP 100 | 4:1 | 0.87% |
| 2 | 98:2 | PEP98 | 4:1 | 0.87% |
| 3 | 96:4 | PEP96 | 4:1 | 0.87% |
| 4 | 94:6 | PEP94 | 4:1 | 0.87% |

The stents were weighed, spray coated with the respective formulations and dried overnight at 40°C under vacuum. Upon weighing the average coating weight of the stents was 442µg yielding a dexamethasone content of 89µg +/- 8µg.

Each stent was placed on a hook and suspended in a vial containing 8ml of an aqueous release medium with a 5mm magnetic flea. The vials were placed on a multipoint stirrer set at 1000rpm and incubated at 37°C.

After a given time interval (ranging from 1 hour to several days), the stents were transferred to fresh aliquots of release medium and replaced on the stirrer to resume agitation. The solution containing dexamethasone was set aside for analysis.

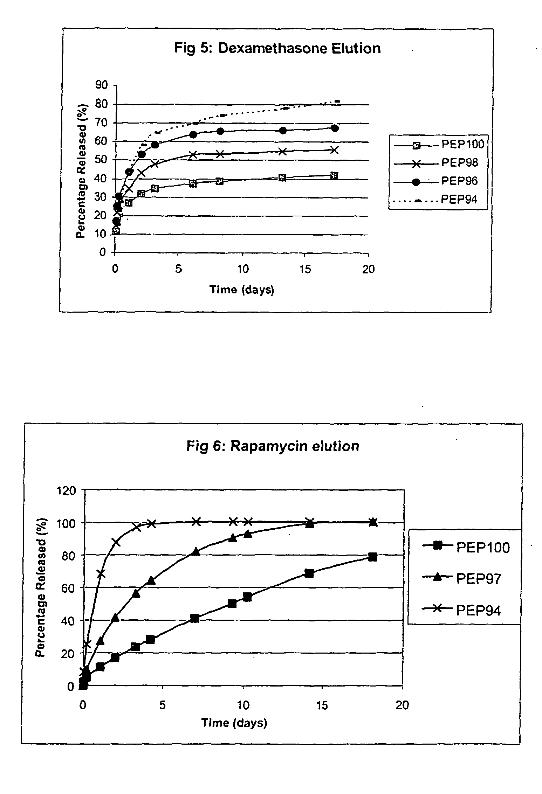

Analysis of drug content was carried out using a UV spectrophotometer (CamSpec M350) at 241nm. The concentration in each sample was determined from a standard curve of absorbance at 241nm versus concentration of calibration solutions (see Fig. 5).

### Example 2: Rapamycin

This example describes the preparation and elution of stents comprising various ratios of PVB/PnVPA and Rapamycin

Stents were cleaned by immersion in IPA for 30 mins with ultrasound and dried at 100°C overnight.

Four coating solutions were prepared in chloroform comprising various ratios of PVB/PnVPA (Table 2). Rapamycin was added to each formulation so that in terms of total solids 25% was rapamycin, 75% polymer.

**Table 2**

| Coating Solution | Ratio PVB/PnVPA | Nomenclature | Ratio Polymer: Drug | Coating Solution Total Solids |
|---|---|---|---|---|
| 1 | 100:0 | PEP 100 | 3:1 | 1% |
| 2 | 97:3 | PEP97 | 3:1 | 1% |
| 3 | 94:6 | PEP94 | 3:1 | 1 % |

The stents were weighed, spray coated with the respective formulations and dried overnight at 40C under vacuum. Upon weighing the average coating weight of the stents was 755µg yielding a rapamycin content of 189µg +/- 13µg.

Each stent was placed on a hook and suspended in a vial containing 8ml of an aqueous release medium with a 5mm magnetic flea. The vials were placed on a multipoint stirrer set at 1000rpm and incubated at 37°C.

After a given time interval (ranging from 1 hour to several days), the stents were transferred to fresh aliquots of release medium and replaced on the stirrer to resume agitation. The solution containing rapamycin was set aside for analysis.

Analysis of drug content was carried out using a UV spectrophotometer (CamSpec M350) at a wavelength of 279nm. The concentration in each sample was determined from a standard curve of absorbance at 279nm versus concentration of calibration solutions (see Fig. 6).

### Example 3: 17β-Estradiol

This example describes the preparation and elution of stents comprising various ratios of PVB/PnVPA and 17β-Estradiol

Stents were cleaned by immersion in IPA for 30 mins with ultrasound and dried at 100°C overnight.

Three coating solutions were prepared in a blend of chloroform and acetone (80:20 by weight) comprising various ratios of PVB/PnVPA (Table 3). 17β-Estrradiol was added to each formulation so that in terms of total solids 25% was 17β-Estradiol, 75% polymer.

**Table 3**

| Coating Solution | Ratio PVB/PnVPA | Nomenclature | Ratio Polymer: Drug | Coating Solution Total Solids |
|---|---|---|---|---|
| 1 | 94:6 | PEP94 | 3:1 | 1% |
| 2 | 90:10 | PEP90 | 3:1 | 1% |
| 3 | 86:14 | PEP86 | 3:1 | 1% |

The stents were weighed, spray coated with the respective formulations and dried overnight at 40°C under vacuum. Upon weighing the average coating weight of the stents was 904µg yielding a 17β-Estradiol content of 229µg +/- 32µg.

Each stent was placed on a hook and suspended in a vial containing 8ml of an aqueous release medium with a 5mm magnetic flea. The vials were placed on a multipoint stirrer set at 1000rpm and incubated at 37°C.

After a given time interval (ranging from 1 hour to several days), the stents were transferred to fresh aliquots of release medium and replaced on the stirrer to resume agitation. The solution containing 17β-Estradiol was set aside for analysis.

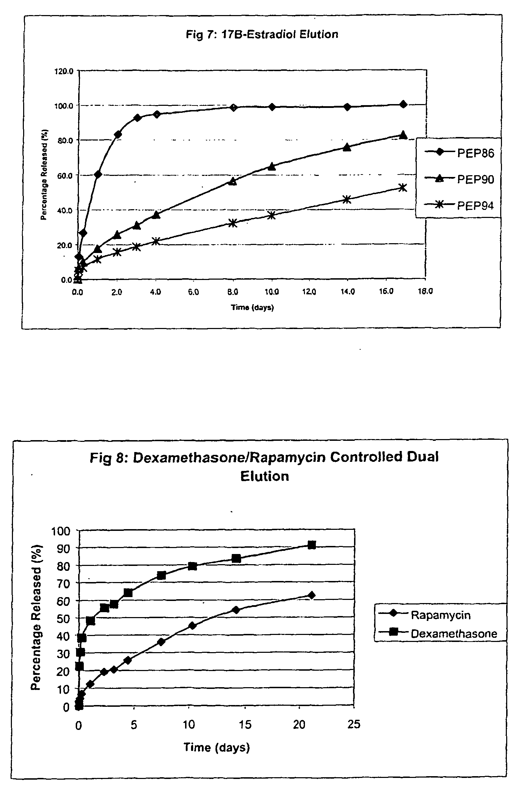

Analysis of drug content was carried out using a UV spectrophotometer (CamSpec M350) at wavelengths of 225 and 281nm. The concentration in each sample was determined from a standard curves of absorbance at 225 and 281nm versus concentration of calibration solutions. Close agreement is obtained between the two wavelengths. Figure 7 shows the results at 281nm.

### Example 4: Dual Elution Dexamethasone/Rapamycin

This example describes the preparation and elution of stents comprising rapamycin and dexamethasone each contained within a specific ratio of PVB/PnVPA

Stents were cleaned by immersion in IPA for 30 mins with ultrasound and dried at 100°C overnight.

A coating solution was prepared made up of PEP100 and rapamycin in chloroform, so that in terms of total solids 25% was rapamycin, 75% polymer.

A second coating solution was prepared made of PEP 94 and dexamethasone in a mixture of chloroform and acetone (80:20 by volume), so that in terms of total solids 25% was dexamethasone, 75% polymer.

**Table 4**

| Coating Solution | Ratio PVB/PnVPA | Nomenclature | Drug | Ratio Polymer: Drug | Coating Solution Total Solids |
|---|---|---|---|---|---|
| 1 | 100:0 | PEP 100 | Rapamycin | 3:1 | 1 % |
| 2 | 94:6 | PEP94 | Dexamethasone | 3:1 | 1% |

The stents were weighed, spray coated in equal amounts with the respective formulations such that the bottom layer comprised PEP 100 with rapamycin and the top layer PEP94 with dexamethasone. The stents were then dried overnight at 40°C under vacuum.

Upon weighing the average coating weight of the stents was 680µg yielding drug loadings of approx 85µg +/- 11µg for each individual drug.

Each stent was placed on a hook and suspended in a vial containing 8ml of an aqueous release medium with a 5mm magnetic flea. The vials were placed on a multipoint stirrer set at 1000rpm and incubated at 37C.

After a given time interval (ranging from 1 hour to several days), the stents were transferred to fresh aliquots of release medium and replaced on the stirrer to resume agitation. The solution containing dexamethasone/rapamycin was set aside for analysis.

Analysis of drug content was carried out using a UV spectrophotometer (CamSpec M350) at 241nm and 291nm.

Due to spectral overlap estimates of absorbance were made, for rapamycin at 291nm where the effect of dexamethasone was minimal and mathematically for dexamethasone through deconvolution of the overall trace.

The concentrations of dexamethasone and rapamycin in each sample were determined from standard curves of absorbance at 241nm and 291nm respectively versus concentration of calibration solutions (see Fig. 8).

### Example 5: Dual Elution Dexamethasone/17β-Estradiol

This example describes the preparation and elution of stents comprising 17β- estradiol and dexamethasone each contained within a specific ratio of PVB/PnVPA

Stents were cleaned by immersion in IPA for 30 mins with ultrasound and dried at 100°C overnight.

A coating solution was prepared made up of PEP94 and 17β-estradiol in a blend of chloroform and acetone (80:20 by weight), so that in terms of total solids 25% was 17β-estradiol , 75% polymer.

A second coating solution was prepared made of PEP 90 and dexamethasone in a mixture of chloroform and acetone (80:20 by weight), so that in terms of total solids 25% was dexamethasone, 75% polymer.

**Table 5**

| Coating Solution | Ratio PVB/PnVPA | Nomenclature | Drug | Ratio Polymer: Drug | Coating Solution Total Solids |
|---|---|---|---|---|---|
| 1 | 94:6 | PEP94 | 17β-Estradiol | 3:1 | 1% |
| 2 | 90:10 | PEP90 | Dexamethasone | 3:1 | 1% |

The stents were weighed, spray coated in a ratio of 3:1 (estradiol: dexamethasone) with the respective formulations such that the bottom layer comprised PEP94 with 17β-estradiol and the top layer PEP90 with dexamethasone. The stents were then dried overnight at 40°C under vacuum.

Upon weighing the average coating weight of the stents was 811 µg yielding drug loadings of approx 147µg +/- 17µg for 17β-estradiol and 57µg +/- 16ug for dexamethasone.

Each stent was placed on a hook and suspended in a vial containing 8ml of an aqueous release medium with a 5mm magnetic flea. The vials were placed on a multipoint stirrer set at 1000rpm and incubated at 37°C. After a given time interval (ranging from 1 hour to several days), the stents were transferred to fresh aliquots of release medium and replaced on the stirrer to resume agitation. The solution containing dexamethasone/17β-estradiol was set aside for analysis.

Analysis of the samples was carried out using HPLC (Agilent 1100 series).

### Dexamethasone

| Time (Hours) | Total Microgrammes Released (µg) | Percentage Released (%) |
|---|---|---|
| 1 | 28 | 49.1 |
| 7 | 42.9 | 75.3 |
| 24 | 53.8 | 94.3 |

### 17β-estradiol

| Time (Hours) | Total Microgrammes Released (µg) | Percentage Released (%) |
|---|---|---|
| 1 | 7.5 | 5.1 |
| 7 | 15.5 | 10.5 |
| 24 | 25.1 | 17.1 |

### Example 6: Polymer Adhesion

An experiment was carried out to demonstrate the improved adhesion of PEP coating on a functionalised surface and a surface incorporating a primer layer on top of a functionalised surface. Three sets of plate samples were prepared for pull strength testing. Each set contained five plates.
1 Unfunctionalised steel / no primer / PEP94 coating dried at 40°C
2 Functionalised steel / no primer / PEP94 coating dried at 40°C
3 Functionalised steel / PEP100 primer / PEP94 coating dried at 40°C
where PEP100 has a 100% PVB and no PnVPA and PEP94 has PVB and PnVPA in a ratio of 94:6.

### Method

### Sample Preparation

0.25mm thick stainless steel plate was cut to the required size (25 x 50mm), cleaned in 2-propanol, and dried thoroughly.

Plates from sets 2 & 3, were reacted with N-[3-(trimethoxysilyl) propyl] ethylenediamine at 5 % by weight in toluene with a small amount of acetic acid present, followed by drying at 50°C and rinsing with a series of solvent washes (methanol and water) to produce an amine functionalised surface. The surface was further reacted with 3-(triethoxysilyl) propyl isocyanate at 5 % by weight in anhydrous toluene, followed by rinsing with more toluene, and drying to produce a triethoxysilylated surface.

Functionalised plates (set 3) were coated with a primer layer of PEP100 at 1 % by weight in chloroform using an airbrush to apply an even coating of around 1.5 milligrams to one side of each plate. This coating was dried at 100°C for 20 hours.

Untreated set 1, and functionalised set 2 plates were coated with a 1 %w/w PEP94 solution in chloroform, applied by airbrush to give an even coating on one side of the plate of around 15 milligrams. The plates were dried at 40°C under reduced pressure for 16 hours.

Functionalised and primed set 3 plates were coated with a 1%w/w PEP94 solution in chloroform, applied using an airbrush to give an even coating on one side of the plate of around 15 milligrams, in total, including the primer coat. The plates were dried at 40°C under reduced pressure for 16 hours.

The final coating weights for the plates.(including any primer) fell within a range of 12 to 17 milligrams with the average being 14 milligrams.

### Adhesion Strength Testing

Testing was carried out by The National Physics Laboratory (Middlesex, UK) The area of each plate to be tested was soaked with a solution of 0.01 %w/w aqueous sodium dodecyl sulphate for two hours. The sample was then tissue dried and an aluminium stub attached to the area with a high strength adhesive.

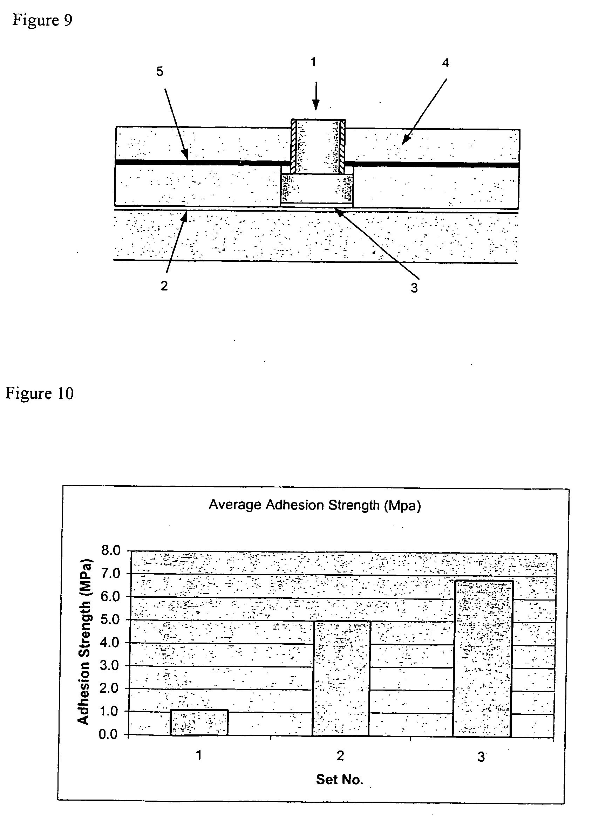

Fig 9 depicts a schematic representation of pull-off test equipment in which aluminium stub 1 is attached to test coating 2 with adhesive bond 3.

The adhesion tests were performed using a commercially available Elcometer Patti 100 test rig with a F20 piston. In testing the stub 1 and top platen 4 are forced apart by the inflation of pneumatic bladder 5. The maximum pneumatic pressure at coating separation was recorded and converted to an adhesion strength using conversion tables supplied with the test rig. In all the samples tested the fracture occurred at the coating - steel interface.

### Results

The average adhesion strength value for each set is shown in Figure 10.

Results show significantly improved adhesion strength for functionalisated samples in comparison to untreated surfaces. Further improvements in adhesion strength are made with the addition of the primer layer.

NB Increasing the drying temperature of set 3 plates from 40°C to 60°C has no effect on adhesion strength.

## Claims

1. A coating composition for an implantable medical device comprising a combination of a bioactive and a vehicle therefor, wherein the vehicle comprises a first compound which is a random copolymer of Formula 1;
[A]ₓ-[B]_{y}-[C]_{z}
wherein A is a vinyl acetal group, B is a vinyl alcohol group and C is a vinyl acetate group and wherein x+y+z=1 and x is from 0.8 to 0.9, y is from 0.1 to 0.2 and z is from 0 to 0.025,
and a second compound which is polymer of Formula 2:
[D]ₙ-[E]ₘ
wherein D is a vinyl pyrrolidone group and E is a vinyl acetate group and wherein n+m=1.

2. A composition as claimed in claim 1, wherein the proportion of said second compound is up to 80% by weight of the coating composition.

3. A composition as claimed in claim 1 or 2, wherein n is from 0.3 to 0.7 and m is from 0.3 to 0.7.

4. A composition as claimed in any preceding claim, wherein said second compound is poly(vinyl pyrrolidone-co-vinyl acetate) with an average Mw of 50,000.

5. A composition as claimed in any preceding claim, wherein [A]ₓ-[B]_{y}-[C]_{z} is a compound of Formula 1A: 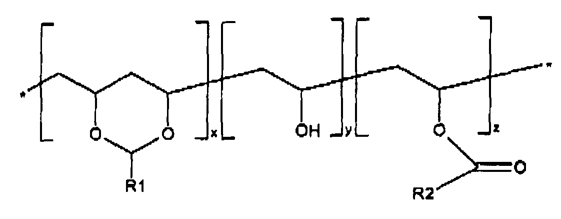 wherein R1 and R2 are independently H or an alkyl, alkenyl, alkynyl or aryl group and
wherein optionally an alkyl, alkenyl, alkynyl or aryl group may be substituted for any pendent hydrogen atom.

6. A composition as claimed in claim 5, wherein said first compound is poly(vinylbutyral-co-vinyl alcohol-co-vinyl acetate) with an average Mw from 50,000 to 80,000 and with 88 wt% vinyl butyral groups.

7. A composition as claimed in any preceding claim, wherein the bioactive is dexamethasone, rapamycin or 17β-Estradiol.

8. A composition as claimed in any preceding claim, wherein the proportion of bioactive to vehicle is from 1:9 to 1:1.

9. A method for coating a medical device comprising the step of:
(a) applying to at least a part of the device a first coating composition as claimed in any of claims 1 to 8.

10. A method as claimed in claim 9 additionally comprising the step of:
(b) applying a second coating composition as claimed in any of claims 1 to 8
wherein said first and said second coating composition are the same or different.

11. A method as claimed in claim 10, wherein the first coating has a vehicle comprising said first compound and said second compound in a ratio from 80:20 to 100:0 and wherein the second coating has a vehicle comprising said first compound and said second compound in a ratio from 70:30 to 94:6.

12. A method as claimed in claim 10, wherein the ratio of said first compound to said second compound is 98:2 in the first coating and 90:10 in the second.

13. A method as claimed in any of claims 10 to 12, wherein the first coating composition includes rapamycin and the second coating composition includes dexamethasone.

14. A medical device having a first coating of a composition as claimed in any of claims 1 to 8, wherein the first coating is applied directly to the device.

15. A device as claimed in claim 14 having a second coating (which is the same as or different to the first coating) as claimed in any of claims 1 to 8, wherein the second coating is applied to at least a part of the first coating.

16. A device as claimed in claim 15 wherein the first coating has a vehicle comprising said first compound and said second compound in a ratio from 80:20 to 100:0 and wherein the second coating has a vehicle comprising said first compound and said second compound in a ratio from 70:30 to 94:6.

17. A device as claimed in claim 16, wherein the ratio of said first compound to said second compound is 98:2 in the first coating and 90:10 in the second.

18. A device as claimed in any of claims 15 to 17, wherein the first coating composition includes rapamycin and the second coating composition includes dexamethasone.

19. A device as claimed in any of claims 14 to 18 which is a stent or graft-stent.

20. A vehicle for carrying a bioactive, wherein the vehicle is as defined in any of claims 1 to 6.

## Patentansprüche

1. Zusammensetzung für eine Beschichtung für ein implantierbares medizinisches Gerät enthaltend eine Kombination eines bioaktiven Materials und eines Vehikels dafür, wobei das Vehikel eine erste Verbindung, die ein statistisches Copolymer der Formel 1 ist:
[A]ₓ-[B]_{y}-[C]_{z}
**wobei** A eine Vinylacetalgruppe, B eine Vinylalkoholgruppe und C eine Vinylacetatgruppe ist und wobei x+y+z=1 und x von 0,8 bis 0,9, y von 0,1 bis 0,2 und z von 0 bis 0,025 gewählt wird,
und eine zweite Verbindung, die ein Polymer der Formel 2 ist:
[D]ₙ-[E]ₘ
wobei D eine Vinylpyrrolydongruppe und E eine Vinylacetatgruppe ist und wobei n+m=1, enthält.

2. Zusammensetzung nach Anspruch 1,
**wobei** der Anteil der zweiten Verbindung bis zu 80 Gewichtsprozent der Zusammensetzung für die Beschichtung beträgt.

3. Zusammensetzung nach Anspruch 1 oder 2,
**wobei** n 0,3 bis 0,7 und m 0,3 bis 0,7 ist.

4. Zusammensetzung nach einem der vorhergehenden Ansprüche,
**wobei** die zweite Verbindung Poly(vinylpyrrolydon-co-vinylacetat) mit einem mittleren Mw von 50.000 ist.

5. Zusammensetzung nach einem der vorhergehenden Ansprüche,
**wobei** [A]ₓ-[B]_{y}-[C]_{z} eine Verbindung der Formel 1A ist: 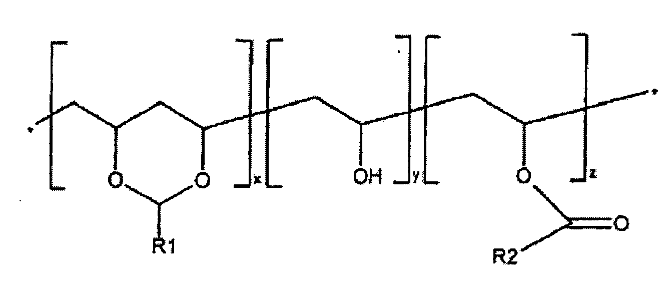 **wobei** R1 und R2 voneinander unabhängig H oder eine Alkyl-, Alkenyl-, Alkynyl- oder Arylgruppe sind und wobei gegebenenfalls eine Alkyl-, Alkenyl-, Alkynyl- oder Arylgruppe jedes beliebige Wasserstoffatom ersetzen können.

6. Zusammensetzung nach Anspruch 5,
wobei die erste Verbindung Poly(vinylbutyral-co-vinyl alkohol-co-vinylacetat) mit einem mittleren Mw von 50.000 bis 80.000 und mit 88 Gewichtsprozent Vinylbutyralgruppen ist.

7. Zusammensetzung nach einem der vorhergehenden Ansprüche,
**wobei** das bioaktive Material Dexamethason, Rapamycin oder 17β-Estradiol ist.

8. Zusammensetzung nach einem der vorhergehenden Ansprüche,
**wobei** der Anteil von bioaktivem Material zu Vehikel 1:9 bis 1:1 ist.

9. Verfahren zur Beschichtung eines medizinischen Gerätes umfassend den Schritt:
(a) Aufbringen auf wenigstens einem Teil des Geräts einer ersten Beschichtungszusammensetzung nach einem der Ansprüche 1 bis 8.

10. Verfahren nach Anspruch 9 zusätzlich umfassend den Schritt:
(b) Aufbringen einer zweiten Beschichtungszusammensetzung nach einem der Ansprüche 1 bis 8, wobei die erste und zweite Beschichtungszusammensetzung gleich oder verschieden sind.

11. Verfahren nach Anspruch 10,
**wobei** die erste Beschichtung ein Vehikel hat, das die erste Verbindung und die zweite Verbindung in einem Verhältnis von 80:20 bis 100:0 enthält und wobei die zweite Beschichtung ein Vehikel hat, das die erste Verbindung und die zweite Verbindung in einem Verhältnis von 70:30 bis 94:6 enthält.

12. Verfahren nach Anspruch 10,
**wobei** das Verhältnis der ersten Verbindung zu der zweiten Verbindung 98:2 in der ersten Beschichtung und 90:10 in der zweiten Beschichtung ist.

13. Verfahren nach einem der Ansprüche 10 bis 12,
wobei die erste Belschichtungszusammensetzung Rapamycin enthält und die zweite Beschichtungszusammensetzung Dexamethason enthält.

14. Ein medizinisches Gerät mit einer ersten Beschichtung mit einer Zusammensetzung nach einem der Ansprüche 1 bis 8,
**wobei** die erste Beschichtung direkt auf das Gerät aufgetragen ist.

15. Gerät nach Anspruch 14 mit einer zweiten Beschichtung (die die gleiche Beschichtung wie die erste Beschichtung oder davon verschieden ist) nach einem der Ansprüche 1 bis 8,
wobei die zweite Beschichtung wenigstens auf einen Teil der ersten Beschichtung aufgetragen ist.

16. Gerät nach Anspruch 15,
**wobei** die erste Beschichtung ein Vehikel hat, das die erste Verbindung und die zweite Verbindung in einem Verhältnis von 80:20 bis 100:0 enthält und
wobei die zweite Beschichtung ein Vehikel hat, das die erste Verbindung und die zweite Verbindung in einem Verhältnis von 70:30 bis 94:6 hat.

17. Gerät nach Anspruch 16,
**wobei** das Verhältnis der ersten Verbindung zu der zweiten Verbindung 98:2 in der ersten Beschichtung und 90:10 in der zweiten Beschichtung beträgt.

18. Gerät nach einem der Ansprüche 15 bis 17,
**wobei** die erste Beschichtungszusammensetzung Rapermycin enthält und die zweite Beschichtungszusammensetzung Dexamethason enthält.

19. Gerät nach einem der Ansprüche 14 bis 18,
wobei das Gerät ein Stent oder ein Graft-Stent ist.

20. Vehikel für ein bioaktives Material,
**wobei** das Vehikel ein Vehikel ist, wie es in einem der Ansprüche 1 bis 6 definiert ist.

## Revendications

1. Composition de revêtement pour un dispositif médical implantable comprenant une combinaison d'un agent bioactif et d'un véhicule pour ce faire, dans laquelle le véhicule comprend un premier composé qui est un copolymère aléatoire de formule 1 :
[A]ₓ-[B]_{y}-[C]_{z}
dans laquelle A est un groupe acétal vinylique, B est un groupe alcool vinylique et C est un groupe acétate vinylique et dans laquelle x + y + z = 1 et x est compris entre 0,8 et 0,9, y est compris entre 0,1 et 0,2 et z est compris entre 0 et 0,025,
et un second composé qui est un polymère de formule 2 :
[D]ₙ-[E]ₘ
dans laquelle D est un groupe vinyl pyrrolidone et E est un groupe acétate vinylique et dans laquelle n + m = 1.

2. Composition telle que revendiquée dans la revendication 1, dans laquelle la proportion dudit second composé peut atteindre jusqu'à 80 % en poids de la composition de revêtement.

3. Composition telle que revendiquée dans la revendication 1 ou 2, dans laquelle n est compris entre 0,3 et 0,7 et m est compris entre 0,3 et 0,7.

4. Composition telle que revendiquée dans l'une quelconque des revendications précédentes, dans laquelle ledit second composé est un poly(vinyl pyrrolidone-co-acétate vinylique) avec un poids moléculaire moyen de 50 000.

5. Composition telle que revendiquée dans l'une quelconque des revendications précédentes, dans laquelle [A]ₓ-[B]_{y}-[C]_{z} est un composé de formule 1A : 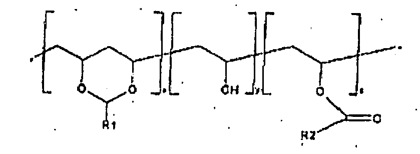 dans laquelle R1 et R2 sont indépendamment H ou un groupe alkyle, alcényle, alcynyle ou aryle et dans laquelle éventuellement un groupe alkyle, alcényle, alcynyle ou aryle peut remplacer n'importe quel atome d'hydrogène pendant.

6. Composition telle que revendiquée dans la revendication 5, dans laquelle ledit premier composé est du poly(vinylbutyral-co-alcool vinylique-co-acétate vinylique) avec un poids moléculaire moyen compris entre 50 000 et 80 000 et avec 88 % en poids de groupes de vinylbutyral.

7. Composition telle que revendiquée dans l'une quelconque des revendications précédentes, dans laquelle l'agent bioactif est de la dexaméthasone, de la rapamycine ou du 17β-estradiol.

8. Composition telle que revendiquée dans l'une quelconque des revendications précédentes, dans laquelle la proportion d'agent bioactif sur le véhicule est comprise entre 1/9 et 1/1.

9. Procédé de revêtement d'un dispositif médical comprenant l'étape consistant à :
(a) appliquer sur au moins une partie du dispositif une première composition de revêtement telle que revendiquée dans l'une quelconque des revendications 1 à 8.

10. Procédé tel que revendiqué dans la revendication 9 comprenant en outre l'étape consistant à :
(b) appliquer une seconde composition de revêtement telle que revendiquée dans l'une quelconque des revendications 1 à 8, dans laquelle ladite première et ladite seconde composition de revêtement sont les mêmes ou sont différentes.

11. Procédé tel que revendiqué dans la revendication 10, dans lequel le premier revêtement a un véhicule comprenant ledit premier composé et ledit second composé dans un rapport compris entre 80/20 et 100/0 et dans lequel le second revêtement a un véhicule comprenant ledit premier composé et ledit second composé dans un rapport compris entre 70/30 et 94/6.

12. Procédé tel que revendiqué dans la revendication 10, dans lequel le rapport dudit premier composé sur ledit second composé est 98/2 dans le premier revêtement et 90/10 dans le second.

13. Procédé tel que revendiqué dans l'une quelconque des revendications 10 à 12, dans lequel la première composition de revêtement inclut de la rapamycine et la seconde composition de revêtement inclut de la dexaméthasone.

14. Dispositif médical ayant un premier revêtement d'une composition telle que revendiquée dans l'une quelconque des revendications 1 à 8, dans lequel le premier revêtement est appliqué directement sur le dispositif.

15. Dispositif tel que revendiqué dans la revendication 14 ayant un second revêtement (qui est le même que le premier revêtement ou qui est différent de celui-ci) tel que revendiqué dans l'une quelconque des revendications 1 à 8, dans lequel le second revêtement est appliqué sur au moins une partie du premier revêtement.

16. Dispositif tel que revendiqué dans la revendication 15, dans lequel le premier revêtement a un véhicule comprenant ledit premier composé et ledit second composé dans un rapport compris entre 80/20 et 100/0 et dans lequel le second revêtement a un véhicule comprenant ledit premier composé et ledit second composé dans un rapport compris entre 70/30 et 94/6.

17. Dispositif tel que revendiqué dans la revendication 16, dans lequel le rapport dudit premier composé sur ledit second composé est 98/2 dans le premier revêtement et 90/10 dans le second.

18. Dispositif tel que revendiqué dans l'une quelconque des revendications 15 à 17, dans lequel la première composition de revêtement inclut de la rapamycine et la seconde composition de revêtement inclut de la dexaméthasone.

19. Dispositif tel que revendiqué dans l'une quelconque des revendications 14 à 18 qui est un stent ou un stent greffon.

20. Véhicule de transport d'un agent bioactif, dans lequel le véhicule est tel que défini dans l'une quelconque des revendications 1 à 6.
